# FASCICULE DE BREVET EUROPEEN

(11) **EP 0 658 098 B1**
(45) Date de publication et mention de la délivrance du brevet: **11.03.1998**
(21) Numéro de dépôt: 94917694.5
(22) Date de dépôt: 01.06.1994
(51) Int. Cl.: A61K 7/42, A61K 7/48, A61K 7/027, A61K 7/043, A61K 7/075, A61K 7/13, A61K 7/16, A61K 7/32, A61K 7/09, C07C 317/18

(54) **COMPOSITIONS COSMETIQUES COMPORTANT DES COMPOSES HYDRO-FLUORO-CARBONES**
HYDROFLUORKOHLENSTOFF-VERBINDUNGEN ENTHALTENDE KOSMETISCHE ZUBEREITUNGEN
COSMETIC COMPOSITIONS COMPRISING HYDROFLUOROCARBON-CONTAINING COMPOUNDS

(30) Priorité: 02.06.1993 FR 9306604
(43) Date de publication de la demande: 21.06.1995
(73) Titulaire: L'OREAL, 75008 Paris (FR)
(72) Inventeur: BOLLENS, Eric, F-94410 Saint-Maurice (FR); MAHIEU, Claude, F-75017 Paris (FR)
(74) Mandataire: Casalonga, Axel
(86) Numéro de dépôt international: FR9400633
(87) Numéro de publication internationale: WO9427568

(56) Documents cités:
- WO-A-93/11103
- FR-A- 2 416 222

## Description

La présente invention concerne l'utilisation de composés hydro- et fluorocarbonés dans des compositions cosmétiques, les compositions cosmétiques en comportant ainsi que certains composés hydro- et fluoro-carbonés du type sulfoxyde ou sulfone.

Il est connu d'utiliser des perfluoropolyéthers, notamment dans des procédés de nettoyage, de protection, de maquillage de la peau ou bien de lavage des cheveux. Ces composés sont connus pour leur basse tension superficielle et leur facilité d'étalement, mais présentent une solubilité dans la plupart des fluides très réduite, excepté dans les fluides fluorés, ce qui rend très difficile leur formulation dans des compositions cosmétiques. Certains de ces composés, les perfluorométhylisopropyléthers, sont connus sous le nom de "FOMBLIN HC", commercialisés par la Société MONTEFLUO.

La demanderesse a maintenant découvert des composés qui, contrairement aux FOMBLIN, présentent une bonne solubilité, notamment dans les solvants classiques utilisés en cosmétique comme les alcools inférieurs, ainsi que les corps gras et huiles usuels. Leur caractère amphiphile, leurs propriétés et leur compatibilité avec les solvants permettent notamment de préparer des compositions homogènes et stables, et par exemple d'assurer une bonne stabilité des émulsions dans laquelle ils interviennent.

Ainsi, la présente invention concerne l'utilisation de composés de formule :

R_{F} - (CH₂)ₙ - X - [C₃H₅(OH)] - Y - R_{H} (I)

dans laquelle C₃H₅(OH) représente les structures : 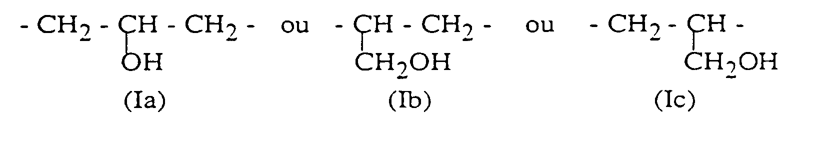
R_{F} représente un radical alkyle perfluoré en C₄-C₂₀ ou un mélange de radicaux alkyle perfluorés en C₄-C₂₀;
R_{H} représente un radical alkyle linéaire ou ramifié en C₁-C₂₂ ou un mélange de radicaux alkyle linéaires ou ramifiés en C₁-C₂₂ ou un radical aryle ou aralkyle;
n est compris entre 0 et 4;
X et Y représentent S, 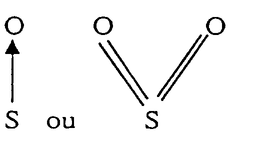
dans des compositions cosmétiques.

Parmi ces composés, les composés préférés sont ceux pour lesquels R_{F} désigne un radical alkyle perfluoré en C₆-C₁₂, R_{H} désigne un radical alkyle linéaire ou ramifié en C₃-C₁₈, un radical aryle en C₆-C₁₀ ou un radical aralkyle en C₇-C₁₅ et n est 2.

De préférence, X et Y sont S.

Parmi les radicaux alkyle linéaires ou ramifiés, on peut citer notamment les radicaux butyle, octyle, 2-éthylhexyle, décyle, dodécyle, 2-butyloctyle, hexadécyle, 2-hexyldécyle, et octadécyle.

Parmi les radicaux aralkyle, on peut citer notamment les radicaux 4-nonylphényle et benzyle, et parmi les radicaux aryle, le radical phényle.

Les composés de formule (I) selon l'invention peuvent être préparés en mettant en oeuvre la réaction d'un composé fluoré à hydrogène acide de formule (II) :

R_{F} - (CH₂)ₙ - S - H (II)

avec un époxyde de formule (III) : 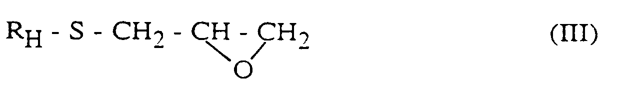 ou la réaction d'un composé hydrocarboné à hydrogène acide de formule (IV) :

R_{H} - S - H (IV)

avec un époxyde fluoré de formule (V) : 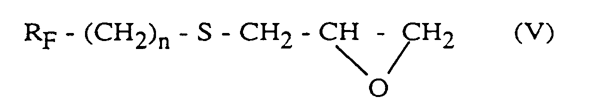 en présence d'un composé basique ou acide jouant le rôle de réactif ou de catalyseur, pour obtenir le composé de formule (I) correspondant, les substituants R_{F}, R_{H} et n ayant dans les formules (II) et (III), (IV) et (V), la même signification que dans la formule (I) et en oxydant éventuellement la fonction mercaptan en sulfoxyde ou sulfone avec un oxydant, de préférence de l'eau oxygénée en milieu acide.

Les composés de formule (V) sont décrits notamment dans le brevet US 3976698 ou dans les demandes de brevet EP 300358 et DE 2018461.

Les composés jouant le rôle de réactif ou de catalyseur peuvent donc être basiques, tels que les métaux alcalins, les hydroxydes de métaux alcalins ou alcalino-terreux, les alcoolates des métaux alcalins tels que les méthylates ou tertiobutylates, les hydrures alcalins tels que l'hydrure de sodium, les amines tertiaires telles que la pyridine ou la triéthylamine. Ils peuvent aussi être des bases de Lewis parmi lesquelles on peut citer les fluorures de césium, de rubidium, de potassium. Ces composés peuvent être également supportés sur un solide tel que l'alumine. De préférence, on utilise un alcoolate alcalin comme le méthylate de sodium ou une amine tertiaire comme la pyridine.

Ces composés peuvent également être acides, notamment lorsque le produit de départ de formule (II) ou (IV) est un alcool. De tels acides peuvent être les acides minéraux ou leurs sels avec des amines tertiaires ou encore des acides dits de Lewis tels que le trifluorure de bore, le tétrachlorure d'étain, le pentachlorure d'antimoine, utilisés seuls, en solution ou associés à un support usuel.

On utilise de préférence un composé basique.

La concentration des composés acides ou basiques jouant le rôle de réactif ou de catalyseur et mis en oeuvre dans la réaction de préparation des composés de formule (I) peut être comprise entre 1 et 100% molaire, de préférence entre 2 et 10% molaire par rapport au composé fluoré à hydrogène acide de formule (II) ou (IV).

La réaction de préparation peut être mise en oeuvre en présence de solvant ou en l'absence de solvant.

Comme solvant, on peut utiliser des hydrocarbures aliphatiques tels que l'heptane, l'hexane ou des hydrocarbures cycliques tels que le cyclohexane, des hydrocarbures aromatiques tels que le toluène, des éthers tels que l'éther éthylique ou isopropylique, des éthers cycliques tels que le dioxane, ou bien encore l'acétonitrile, le diméthylformamide, la N-méthylpyrrolidone, le diméthylacétamide ou encore les alcools tels que le méthanol, l'éthanol ou l'isopropanol.

Pour préparer les composés selon l'invention, on peut mélanger d'abord le composé à hydrogène acide de formule (II) ou (IV) avec le réactif ou catalyseur acide ou basique, sous atmosphère inerte. Pour réaliser le mélange, on peut opérer à une température comprise entre 20 et 180°C, de préférence entre 50 et 150°C. Par atmosphère inerte, on entend par exemple une atmosphère d'azote, d'argon ou d'hélium.

Le mélange peut être effectué, selon la nature du composé à hydrogène acide et du réactif ou catalyseur, en l'absence ou en présence de solvant.

On ajoute au mélange obtenu l'époxyde de formule (III) ou (V), cette addition pouvant s'effectuer en une seule fois ou progressivement, sur une période pouvant aller de 30 minutes à 2 heures par exemple.

Le temps de réaction est alors compris entre environ 1 heure et 24 heures, de préférence entre 1 heure et 3 heures.

Le composé issu de la réaction peut être oxydé lorsque celui-ci contient une fonction mercaptan, en sulfoxyde ou en sulfone, en présence d'eau oxygénée en milieu acide, selon des méthodes connues, notamment dans les demandes de brevet français FR 2 099 092 et FR 2 516 920.

Il peut être en outre nécessaire de neutraliser le mélange obtenu, et on peut séparer le composé synthétisé de façon usuelle, par distillation, par exemple.

Lorsque la réaction de préparation des composés de formule (I) est effectuée en présence de composé basique, elle conduit uniquement à des composés pour lesquels C₃H₅(OH) représente le groupement (Ia). Dans le cas où la réaction est effectuée en présence de composé acide, on peut obtenir un mélange de composés correspondant aux significations (Ia), (Ib) et (Ic) de C₃H₅(OH).

Les composés selon l'invention peuvent se présenter sous forme d'huile ou sous forme solide à la température ambiante.

Les composés de formule (I) où X et Y sont S, sont connus et peuvent être utilisés comme lubrifiants.

Un autre objet de l'invention concerne les composés de formule (I') :

R_{F} - (CH₂)ₙ - X - [C₃H₅(OH)] - Y - R_{H} (I')

dans laquelle C₃H₅(OH) représente les structures : 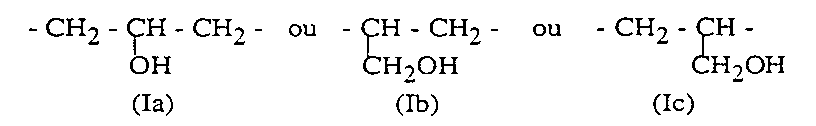
R_{F} représente un radical alkyle perfluoré en C₄-C₂₀ ou un mélange de radicaux alkyle perfluorés en C₄-C₂₀;
R_{H} représente un radical alkyle linéaire ou ramifié en C₁-C₂₂ ou un mélange de radicaux alkyle linéaires ou ramifiés en C₁-C₂₂ ou un radical aryle ou aralkyle;
n est compris entre 0 et 4;
X et Y représentent

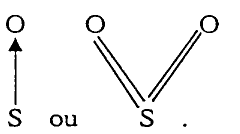

De façon générale, ces composés utilisés dans des compositions cosmétiques permettent d'en améliorer les propriétés cosmétiques. Ils confèrent de la douceur, de la brillance et un toucher non collant aux matières kératiniques telles que la peau, les cheveux et les ongles. Par ailleurs, certains de ces composés se présentant sous forme d'huile incolore, ils peuvent permettre d'obtenir des émulsions transparentes.

C'est pourquoi la présente invention concerne également des compositions cosmétiques comportant au moins un composé de formule (I) et au moins un adjuvant cosmétique .

Ces compositions peuvent se présenter sous forme d'émulsions, de laits ou de crèmes, de lotions huileuses ou oléoalcooliques, sous forme de gels gras ou oléoalcooliques, de dispersions vésiculaires à base de lipides amphiphiles ioniques ou non-ioniques, de bâtonnets solides, de pâte, de spray ou de mousse aérosol.

Selon la forme des compositions auxquelles sont incorporés les composés de l'invention, ces compositions comportent par ailleurs les adjuvants et additifs usuels pour la forme choisie.

Plus précisément, ces compositions peuvent être des laits et des crèmes pour les soins de la peau ou des cheveux, des crèmes, lotions ou laits démaquillants, des crèmes, gels, laits ou lotions anti-solaire, des crèmes ou mousses de rasage, des lotions après-rasage, des shampooings ou des après-shampooings, des déodorants corporels, des pâtes dentifrices, des laques, des compositions de coloration capillaire, des compositions pour la déformation permanente des cheveux, des produits de soin des lèvres ou des ongles.

Ces compositions cosmétiques peuvent être également utilisées comme produit de maquillage des cils, des sourcils, des ongles, des lèvres ou de la peau comme les crèmes de traitement de l'épiderme, des fonds de teint, des bâtons de rouge à lèvres, des fards à paupières ou à joues, des eye-liners ou mascara, des vernis à ongles, par exemple.

Selon l'invention, les composés de formule (I) représentent de 0,1 à 25%, et de préférence de 0,1 à 15% du poids total de la composition.

Parmi les adjuvants cosmétiques usuels pour ce genre de composition, peuvent être présents en outre dans les compositions selon l'invention, des corps gras usuels, des solvants organiques, des silicones, les épaississants, les adoucissants, des filtres solaires UV-A ou UV-B ou à bande large, des agents anti-mousses, des agents hydratants, des humectants, des parfums, des conservateurs, des tensio-actifs, des charges, des séquestrants, des émulsionnants, des polymères anioniques, cationiques, non-ioniques, amphotères ou leurs mélanges, des anti-perspirants, des agents alcalinisants, des colorants, des pigments, des agents propulseurs, des anti-oxydants et des anti-radicaux libres.

Plus précisément, comme corps gras, on peut utiliser une huile ou une cire ou leur mélange, des acides gras, des alcools gras, des esters d'acides gras tels que les triglycérides d'acides gras en C₆ à C₁₈, de la vaseline, de la paraffine, de la lanoline, de la lanoline hydrogénée ou acétylée.

Parmi les huiles, on peut citer les huiles minérales, animales, végétales ou les huiles de synthèse, et notamment l'huile de vaseline, de paraffine, de ricin, de jojoba, de sésame, ainsi que les huiles et des gommes de silicone et les isoparaffines.

Parmi les cires animales, fossiles, végétales, minérales ou de synthèse, on peut notamment citer les cires d'abeille, de Caroube, de Candelila, les ozokérites, les cires microcristallines ainsi que les cires et résines de silicone.

Parmi les solvants organiques usuellement utilisés dans les compositions cosmétiques, on peut citer plus précisément les mono-alcools ou polyalcools inférieurs en C₁ à C₆ comme l'éthanol, l'isopropanol, le propylèneglycol, le glycérol, le sorbitol, les cétones telles que l'acétone, les esters tels que l'acétate de butyle ou l'acétate d'éthyle, le toluène, par exemple.

A titre d'agent épaississant, on peut citer les dérivés de cellulose, les dérivés d'acide polyacrylique, les gommes de guar ou de caroube, ainsi que la gomme de xanthane, par exemple.

Parmi les tensio-actifs, on peut citer notamment les tensio-actifs non-ioniques tels que les alkyl(C₈-C₂₄)polyglycosides où le nombre de motif glucoside est compris entre 1 et 15 et les tensio-actifs non-ioniques du type polyglycérolé.

Les alkylpolyglycosides sont notamment les produits vendus sous la dénomination APG, tels que les produits APG 300, APG 350, APG 500, APG 550, APG 625, APG base 10-12; les produits vendus par la Société SEPPIC sous les dénominations TRITON CG 110 et TRITON CG 312.

Les composés polyglycérolés sont les dérivés qui résultent de la condensation de 1 à 10, de préférence de 2 à 6 moles de glycidol par mole d'alcool ou d'alphadiol en C₁₀-C₁₄, de diglycolamide d'acides gras en C₁₂-C₁₈, tels que décrits dans les brevets FR 1 477 048, 2 328 763, 2 091 516, 2 169 787.

Les dispersions vésiculaires de lipides amphiphiles ioniques ou non-ioniques citées ci-dessus, peuvent être préparées selon des procédés usuels dont on trouve une liste non limitative dans "Les liposomes en biologie cellulaire et pharmacologie" Edition INSERM/John Libbery Eurotext, (1987), p. 6 à 18.

Pour les compositions sous forme de pâte dentifrice, on peut utiliser les adjuvants et additifs usuels comme des agents de polissage tels que la silice, des agents actifs comme les fluorures tels que le fluorure de sodium, et éventuellement des agents édulcorants tels que le saccharinate de sodium.

Les exemples suivants sont destinés à illustrer l'invention et ne sauraient être considérés comme limitatifs de sa portée.

### EXEMPLES DE PREPARATION

### EXEMPLE I

### 1-(2'-F-hexyl-éthylthio)3-octylthio-2-propanol

A la température de 25°C et sous courant d'azote, on ajoute 0,61 g d'une solution méthanolique de méthylate de sodium (environ 30% - 5,65 meq g⁻¹) à 10,05 g (0,069 mole) d'octanethiol.

Le mélange est chauffé à 70°C. On évapore sous vide le méthanol présent dans le milieu.

Le 2-F-hexyl-éthylthioglycidyléther (30 g-0,069 mole) est ensuite ajouté goutte-à-goutte en 30 minutes. On maintient la température du mélange entre 60 et 70°C au cours de l'addition de l'époxyde.

A la fin de l'addition, la température est amenée à 25°C.

Le mélange est neutralisé à l'aide de 3,5 ml de HCl Normal.

Le 1-(2'-F-hexyl-éthylthio)3-octylthio-2-propanol est séparé par distillation : Eb = 178°C/66,5 Pa.

On obtient 30 g (75%) d'une huile transparente incolore.

| Analyse élémentaire : | | | | |
|---|---|---|---|---|
| | % C | % H | % S | % F |
| Calculé | 39,18 | 4,67 | 11,01 | 42,40 |
| Mesuré | 39,16 | 4,65 | 10,57 | 42,46 |

### EXEMPLE II

### 1-(2'-F-hexyl-éthylsulfoxy)3-octylsulfoxy-2-propanol

A la température de 5°C, sous agitation, on additionne un mélange eau oxygénée (110 vol)/acide acétique pur (8,5 ml/0,1 ml) à 11,64 g de 1-(2'-F-hexyl-éthylthio)3-octylthio-2-propanol (0,02 mole), en 20 minutes. La réaction est très exothermique et le mélange prend en masse.

Le produit est transféré dans 100 ml d'eau puis filtré après vive agitation sur verre fritté n°4.

Le résidu solide est lavé à l'eau jusqu'à pH=7.

On obtient 12 g (98%) d'une poudre blanche dont le point de fusion est de 145°C. Il s'agit du 1-(2'-F-hexyl-éthylsulfoxy)3-octylsulfoxy-2-propanol.

| Analyse élémentaire : | | | | |
|---|---|---|---|---|
| | % C | % H | % S | % F |
| Calculé | 37,14 | 4,43 | 10,44 | 40,19 |
| Mesuré | 36,77 | 4,39 | 10,43 | 40,00 |

### EXEMPLE III

### 1-(2'-F-hexyl-éthylsulfonyl)3-octylsulfonyl-2-propanol

A la température de 5°C, sous agitation, on additionne 8,7 g (0,015 mole) de 1-(2'-F-hexyl-éthylthio)3-octylthio-2-propanol en solution dans 15 ml d'acide acétique à un mélange eau oxygénée (110 vol)/acide acétique pur/acide sulfurique pur, dans les proportions respectives suivantes : 10 ml/25 ml/20 gouttes.

A la fin de l'addition, le bain de glace est retiré et le mélange est chauffé 3 heures à 80°C.

Lorsque la température atteint 25°C, le produit qui a pris en masse est transféré dans 50 ml d'eau glacée. Après vive agitation, le solide blanc obtenu est filtré sur verre fritté n°4, puis est lavé jusqu'à pH=7.

Le résidu est alors séché à l'étuve à vide à 40°C.

On obtient 9,3 g (96%) d'une poudre blanche qui est le 1-(2'-F-hexyléthylsulfonyl)3-octylsulfonyl-2-propanol.

| Analyse élémentaire : | | | | |
|---|---|---|---|---|
| | % C | % H | % S | % F |
| Calculé | 35,30 | 4,21 | 9,92 | 38,20 |
| Mesuré | 35,29 | 4,21 | 9,62 | 37,79 |

### EXEMPLE IV

### 1-(2'-F-octyl-éthylthio)3-octadécylthio- 2-propanol

A la température de 30°C, sous courant d'azote, on ajoute 2,1 g de tertiobutylate de potassium à 107 g (0,37 mole) d'octadécanethiol puis on ajoute 50 ml d'éthanol.

La température du mélange est portée à 70°C puis le 2-F-octyl-éthylthioéther de glycidyle est ajouté fondu en 2 heures 30 minutes (200 g - 0,37 mole). La température est maintenue entre 60 et 70°C pendant 4 heures après la fin de l'addition de l'époxyde.

Après retour à 25°C, le résidu est repris dans 15 l d'isopropanol à 40°C. Lorsqu'une solution limpide est obtenue, on ajoute 0,1 ml de HCl concentré puis on filtre le précipité formé. Après retour à 25°C, le mélange prend en masse. Le milieu est alors centrifugé de façon à éliminer dans le surnageant les impuretés solubilisées dans l'isopropanol.

Le résidu est alors séché à l'étuve à vide à la température de 40°C.

On obtient 235 g de 1-(2'-F-octyl-éthylthio)3-octadécylthio-2-propanol sous la forme d'une poudre blanche.
Rendement : 75%.
Point de fusion : 74°C.

| Analyse élémentaire : | | | | |
|---|---|---|---|---|
| | % C | % H | % S | % F |
| Calculé | 45,25 | 5,76 | 7,79 | 39,25 |
| Mesuré | 45,43 | 5,89 | 7,47 | 39,50 |

### EXEMPLES DE FORMULATION

### EXEMPLE 1

### Emulsion huile-dans-eau antisolaire

| | |
|---|---|
| - Mélange d'alcool cétylstéarylique et d'alcool cétylstéarylique oxyéthyléné à 33 moles d'oxyde d'éthylène (80/20), vendu sous la dénomination "DEHSCONET 390" par la Société TENSIA | 7 g MA |
| - Stéarate de glycéryle vendu sous la dénomination "GELEOL COPEAUX" par la Société GATTEFOSSE | 2 g |
| - Huile de silicone vendue sous la dénomination "SILBIONE HUILE 70 047 V300" par la Société RHONE POULENC | 1,5 g |
| - Alcool cétylique | 1,5 g |
| - Huile de vaseline | 15 g |
| - Composé de l'exemple I | 0,1 g |
| - Glycérol | 20 g |
| - Conservateurs qs | |
| - Acide benzène 1,4-di(3-méthylidène-10-campho sulfonique) | 4 g |
| - Eau déminéralisée stérilisée qsp | 100 g |

On réalise l'émulsion en additionnant la phase grasse A portée aux environs de 80°C à la phase aqueuse B portée à la même température et sous agitation rapide. On obtient une émulsion huile-dans-eau se présentant sous la forme d'une crème.

### EXEMPLE 2

### Crème de soin - Emulsion huile-dans-eau

On prépare une crème de soin sous forme d'émulsion huile-dans-eau, ayant la composition suivante :

### Phase A

| | |
|---|---|
| - Tensio-actif non-ionique de type hydroxypropyl-éther obtenu par condensation, en catalyse alcaline, de 3,5 moles de glycidol sur un mélange d'alphadiols en C₁₁-C₁₄, selon le brevet français n° 2.091.516 | 2,4 g |
| - Parahydroxybenzoate de méthyle | 0,2 g |
| - Glycérine | 5 g |
| - Eau | 22,06 g |

### Phase A'

| | |
|---|---|
| - Gel de polyacrylate de glycéryle dans l'eau (50/50), vendu sous la dénomination "HISPAGEL 100" par la Société HISPANO CHEMICA | 56,14 g |

### Phase B

| | |
|---|---|
| - Alcool cétylique | 1 g |
| - Composé de l'exemple II | 5 g |
| - Huile amandes d'abricots | 5 g |
| - Huile de sésame | 1,5 g |
| - Triglycéride d'acide caprylique/caprique, vendu sous la dénomination "MIGLYOL 812" par la Société HULS | 1,5 g |
| - Parahydroxybenzoate de propyle | 0,2 g |

### Mode opératoire :

On chauffe la phase A à 80°C. On ajoute, sous agitation, la phase A' puis la phase B préalablement chauffée à 80°C. On laisse refroidir à température ambiante tout en maintenant l'agitation.

### EXEMPLE 3

### Crème de soin - Emulsion huile-dans-eau

On prépare une crème de soin sous forme d'émulsion huile-dans-eau, ayant la composition suivante :

### Phase A

| | |
|---|---|
| - Tensio-actif non-ionique de type hydroxypropyl-éther obtenu par condensation, en catalyse alcaline, de 3,5 moles de glycidol sur un mélange d'alpha-diols en C₁₁-C₁₄, selon le brevet français n° 2.091.516 | 2,4 g |
| - Parahydroxybenzoate de méthyle | 0,2 g |
| - Glycérine | 5 g |
| - Eau | 22,06 g |

### Phase A'

| | |
|---|---|
| - Gel de polyacrylate de glycéryle dans l'eau (50/50), vendu sous la dénomination "HISPAGEL 100" par la Société HISPANO CHEMICA | 56,14 g |

### Phase B

| | |
|---|---|
| - Alcool cétylique | 1 g |
| - Composé de l'exemple III | 5 g |
| - Huile amandes d'abricots | 5 g |
| - Huile de sésame | 1,5 g |
| - Triglycéride d'acide caprylique/caprique, vendu sous la dénomination "MIGLYOL 812" par la Société HULS | 1,5 g |
| - Parahydroxybenzoate de propyle | 0,2 g |

### Mode opératoire :

On chauffe la phase A à 80°C. On ajoute, sous agitation, la phase A' puis la phase B préalablement chauffée à 80°C. On laisse refroidir à température ambiante tout en maintenant l'agitation.

### EXEMPLE 4

### Crème de soin - Emulsion huile-dans-eau

On prépare une crème de soin sous forme d'émulsion huile-dans-eau, ayant la composition suivante :

### Phase A

| | |
|---|---|
| - Tensio-actif non-ionique de type hydroxypropyl-éther obtenu par condensation, en catalyse alcaline, de 3,5 moles de glycidol sur un mélange d'alpha-diols en C₁₁-C₁₄, selon le brevet français n° 2.091.516 | 2,4 g |
| - Parahydroxybenzoate de méthyle | 0,2 g |
| - Glycérine | 5 g |
| - Eau | 22,06 g |

### Phase A'

| | |
|---|---|
| - Gel de polyacrylate de glycéryle dans l'eau (50/50), vendu sous la dénomination "HISPAGEL 100" par la Société HISPANO CHEMICA | 56,14 g |

### Phase B

| | |
|---|---|
| - Alcool cétylique | 1 g |
| - Composé de l'exemple IV | 5 g |
| - Huile amandes d'abricots | 5 g |
| - Huile de sésame | 1,5 g |
| - Triglycéride d'acide caprylique/caprique, vendu sous la dénomination "MIGLYOL 812" par la Société HULS | 1,5 g |
| - Parahydroxybenzoate de propyle | 0,2 g |

### Mode opératoire :

On chauffe la phase A à 80°C. On ajoute, sous agitation, la phase A' puis la phase B préalablement chauffée à 80°C. On laisse refroidir à température ambiante tout en maintenant l'agitation.

### EXEMPLE 5

### Rouge à lèvres

On prépare un bâton de rouge à lèvres ayant la composition suivante :

| | |
|---|---|
| - Composé de l'exemple I | 1 g |
| - Ozokérite | 14,90 g |
| - Cire microcristalline | 4,90 g |
| - Cire de Candellila | 7,40 g |
| - Huile de Jojoba | 6,20 g |
| - Huile de ricin | 1,20 g |
| - Lanoline liquide | 18,60 g |
| - Lanoline acétylée | 9,90 g |
| - Huile de vaseline | 11,10 g |
| - Talc | 3,70 g |
| - Micatitane | 8,70 g |
| - D & C Red n° 7 Ca lake | 5,20 g |
| - D & C Red n° 7 Ba lake | 2,80 g |
| - FD & C Yellow n° 5 | 1 g |
| - Dioxyde de titane | 3,10 g |
| - Butylhydroxytoluène | 0,30 g |
| - Parfum qs | |

en mélangeant les huiles à une température de 50 à 60°C. Les pigments et laques organiques sont broyés dans la phase huileuse.

On ajoute alors les cires fondues, le talc et le micatitane, puis le parfum.

La composition est alors coulée dans un moule.

L'application du rouge à lèvres est aisée (glisse facilement) et celui-ci confère de la douceur aux lèvres.

Le composé de l'exemple I peut être remplacé par le composé de l'exemple IV.

### EXEMPLE 6

### Vernis à ongles

On prépare un vernis à ongles ayant la composition suivante :

| | |
|---|---|
| - Composé de l'exemple I | 1 g |
| - Nitrocellulose | 10,75 g |
| - Résine toluène sulfonamide formaldéhyde, vendue sous la dénomination "KETJENFLEX MS80" par la Société AKZO | 9,70 g |
| - Acétylcitrate de tributyle vendu sous la dénomination "CITROFLEX A4" par la Société PFIZER | 6,45 g |
| - Toluène | 30,70 g |
| - Acétate de butyle | 21,50 g |
| - Acétate d'éthyle | 9,20 g |
| - Alcool isopropylique | 7,70 g |
| - Acide citrique | 0,05 g |
| - Stéaralkonium hectorite | 1,45 g |
| - Pigments | 1,50 g |

L'étalement du vernis sur l'ongle est aisé et on obtient un film présentant une bonne adhésion sur l'ongle ainsi qu'une bonne brillance. On observe également une bonne tenue de la brillance dans le temps.

### EXEMPLE 7

### Shampooing

| | |
|---|---|
| - Lauryléther carboxylate de sodium (C₁₂/C₁₄ 70/30) oxyéthyléné à 4,5 moles d'oxyde d'éthylène en solution aqueuse à 22%, vendu sous la dénomination "AKYPOSOFT" par la Société CHEM-Y | 7,7 g MA |
| - Lauryléther sulfate de sodium (C₁₂/C₁₄ 70/30) oxyéthyléné à 2,2 moles d'oxyde d'éthylène en solution aqueuse à 28%, vendu sous la dénomination "EMPICOL ESB/3 FL" par la Société MARCHON | 8,4 g MA |
| - Hydroxyéthylcellulose réticulé à l'épichlorhydrine quaternisé par la triméthylamine, vendu sous la dénomination "CELQUAT SC 240" par la Société NATIONAL STARCH | 0,95 g |
| - Monoisopropanolamide d'acide de coprah | 3 g |
| - Suif oxyéthyléné à 60 moles d'oxyde d'éthylène éther de myristylglycol (PM 3000), vendu sous la dénomination "ELFACOS GT 282S" par la Société AKZO | 0,5 g |
| - Composé de l'exemple I | 1 g |
| - Conservateur, parfum | |
| - Eau qsp | 100 g |

Le pH est ajusté à 6 par l'acide chlorhydrique.

### EXEMPLE 8

### Shampooing

| | |
|---|---|
| - Alkyl(C₉/C₁₀/C₁₁ 20/40)polyglycoside 1,4 en solution à 50%, vendu sous la dénomination "APG 300" par la Société HENKEL | 10 g MA |
| - Lauryléthersulfate de sodium (C₁₂/C₁₄ 70/30) oxyéthyléné à 2,2 moles d'oxyde d'éthylène en solution à 28%, vendu sous la dénomination "EMPICOL ESB/3FL" par la Société MARCHON | 5 g MA |
| - Monoisopropanolamide d'acide de coprah | 3 g |
| - Mélange d'alcool cétylstéarylique et d'alcool cétylstéarylique oxyéthyléné à 33 moles d'oxyde d'éthylène, vendu sous la dénomination "DEHSCONET 390" par la Société TENSIA | 2,5 g |
| - Composé de l'exemple IV | 0,5 g |
| - Conservateurs, parfum, colorants | |
| - Eau qsp | 100 g |

Le pH est ajusté à 7 par l'acide chlorhydrique.

### EXEMPLE 9

### Lotion de coiffage

| | |
|---|---|
| - Copolymère méthylvinyléther/monomaléate de butyle à 50% dans l'éthanol, vendu sous la dénomination "GANTREZ ES 425" par la Société ISP neutralisé à 100% par l'amino-2 méthyl-2 propanol-1 | 1 g MA |
| - Composé de l'exemple I | 0,1 g |
| - Ethanol absolu | 52 g |
| - Eau qsp | 100 g |

### EXEMPLE 10

### Coloration capillaire directe

| | |
|---|---|
| -N-(β-hydroxyéthyl)amino-1-nitro-2 N,N'-bis-(β-hydroxyéthyl)amino-4-benzène | 1 g |
| - (nitro-4 méthylamino-3)phénoxyéthanol | 0,1 g |
| - Monochlorhydrate de N',N'-bis-(β-hydroxyéthyl) amino-4-nitro 2-N-(γ-hydroxypropyl)amino-1-benzène | 0,7 g |
| - 4-amino, 2'-méthyl,4'-[N,N-bis-(β-hydroxyéthyl) amino]phénylazobenzène | 0,1 g |
| - 3-nitro 4-β-hydroxyéthylaminophénol | 0,1 g |
| - Diéthanolamide oléique | 3 g |
| - Acide laurique | 1 g |
| - 2-éthoxyéthanol | 5 g |
| - Hydroxyéthylcellulose vendu sous la dénomination "CELLOSIZE WPO 3H" par la Société UNION CARBIDE | 2 g |
| - Composé de l'exemple I | 0,5 g |
| - Amino-2 méthyl-2 propanol-1 qs pH=9,5 | |
| - Eau qsp | 100 g |

La composition est appliquée sur des cheveux gris à 90% de blancs permanentés pendant 30 minutes. Les cheveux sont ensuite rincés, lavés, rincés à nouveau, puis séchés. Les cheveux sont teints en une nuance châtain foncé violine.

### EXEMPLE 11

On prépare une pâte dentifrice en effectuant les mélanges des ingrédients suivants :

| | |
|---|---|
| - Composé de l'exemple I | 0,5 g |
| - Laurylsulfate de sodium en poudre vendu sous la dénomination "EMPICOL LZV/E" par la Société MARCHON à 93% de MA | 1,5 g MA |
| - Alumine hydratée vendue sous la dénomination "ALUMINE SH 100" par la Société SOCHALU | 48 g |
| - Gomme de xanthane vendue sous la dénomination "RHODICARE S" par la Société RHONE POULENC | 1,2 g |
| - Oxyde de titane | 1 g |
| - Sorbitol en solution aqueuse à 70% de MA | 7 g MA |
| - Glycérine | 8 g |
| - Fluorure de sodium | 0,22 g |
| - Parahydroxybenzoate de méthyle | 0,2 g |
| - Saccharinate de sodium | 0,15 g |
| - Arôme qs | |
| - Eau qsp | 100 g |

### EXEMPLE 12

### Stick antitranspirant

| | |
|---|---|
| - Composé de l'exemple IV | 1 g |
| - Alcool stéarylique | 20 g |
| - Myristate d'isopropyle | 10 g |
| - Talc | 1 g |
| - Silice pyrogénée vendue sous la dénomination "AEROSIL R 972" par la Société DEGUSSA | 0,5 g |
| - Chlorure d'aluminium vendu sous la dénomination "REACH 501" par la Société REHEIS | 15 g |
| - Parfum qs | |
| - Cyclométhicone vendu sous la dénomination "DC 245 FLUID" par la Société DOW CORNING qsp | 100 g |

### EXEMPLE 13

### Composition de permanente

On prépare une composition réductrice de déformation permanente des cheveux en procédant au mélange des ingrédients suivants :

| | |
|---|---|
| - Acide thioglycolique | 9 g |
| - Alcool oléique oxyéthyléné à 20 moles d'oxyde d'éthylène, vendu sous la dénomination "BRIJ 98" par la Société ICI | 2 g |
| - Composé de l'exemple I | 0,2 g |
| - Ammoniaque qs pH=8,2 | |
| - Eau déminéralisée qsp | 100 g |

Cette composition est appliquée sur des cheveux mouillés préalablement enroulés sur des rouleaux. Après avoir laissé agir la composition pendant 15 minutes, on rince abondamment à l'eau, puis on applique la composition oxydante suivante :

| | |
|---|---|
| - Eau oxygénée qs 8 volumes | |
| - Alcool oléique oxyéthyléné à 20 moles d'oxyde d'éthylène, vendu sous la dénomination "BRIJ 98" par la Société ICI | 0,5 g |
| - Composé de l'exemple I | 0,1 g |
| - Acide phosphorique qs pH=3,5 | |
| - Eau déminéralisée qsp | 100 g |

On laisse agir la composition oxydante pendant environ 5 minutes, puis on enlève les rouleaux et on rince abondamment la chevelure à l'eau. Après séchage sous casque, les cheveux présentent de belles boucles.

## Revendications

1. Utilisation des composés de formule :
R_{F} - (CH₂)ₙ - X - [C₃H₅(OH)] - Y - R_{H} (I)
dans laquelle C₃H₅(OH) représente les structures : 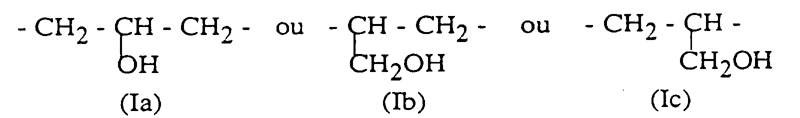
R_{F} représente un radical alkyle perfluoré en C₄-C₂₀ ou un mélange de radicaux alkyle perfluorés en C₄-C₂₀;
R_{H} représente un radical alkyle linéaire ou ramifié en C₁-C₂₂ ou un mélange de radicaux alkyle linéaires ou ramifiés en C₁-C₂₂ ou un radical aryle ou aralkyle;
n est compris entre 0 et 4;
X et Y représentent S, 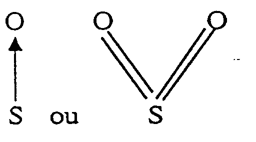
pour la préparation de compositions cosmétiques.

2. Utilisation selon la revendication 1, caractérisée en ce que, dans la formule (I),
R_{F} représente un radical alkyle perfluoré en C₆-C₁₂;
R_{H} représente un radical alkyle linéaire ou ramifié en C₃-C₁₈, un radical aryle en C₆-C₁₀ ou un radical aralkyle en C₇-C₁₅,
n est 2, et
X et Y représentent S.

3. Utilisation selon la revendication 1 ou 2, caractérisée en ce que les compositions cosmétiques sont sous forme de lait, crème, lotion huileuse ou oléoalcoolique, gel gras ou oléoalcoolique, dispersion vésiculaire à base de lipides amphiphiles ioniques ou non-ioniques, bâtonnet solide, pâte, spray ou mousse aérosol.

4. Utilisation selon l'une des revendications 1 à 3, caractérisée en ce que les compositions cosmétiques sont sous forme de lait, de crème de soin pour la peau ou les cheveux, de crème, lotion ou lait démaquillant, de crème, gel, lotion ou lait anti-solaire, de crème ou mousse de rasage, de lotion après-rasage, de shampooing ou d'après-shampooing, de déodorant corporel, de pâte dentifrice, de laque, de composition de coloration capillaire, de composition pour la déformation permanente des cheveux, de produit de maquillage des cils, sourcils, ongles, lèvres ou peau, de crème de traitement de l'épiderme, de fond de teint, de bâton de rouge à lèvres, de fard à paupières ou à joues, d'eye-liner, mascara ou de produit de soin pour les lèvres ou les ongles.

5. Utilisation selon l'une des revendications 1 à 4, caractérisée en ce que les compositions cosmétiques comportent 0,1 à 25% en poids du composé de formule (I).

6. Utilisation selon l'une des revendications 1 à 5, caractérisée en ce que les compositions cosmétiques comportent un ou plusieurs adjuvants choisis dans le groupe formé des corps gras usuels, des solvants organiques, des silicones, les épaississants, les adoucissants, des filtres solaires UV-A ou UV-B ou à bande large, des agents anti-mousses, des agents hydratants, des humectants, des parfums, des conservateurs, des tensio-actifs, des charges, des séquestrants, des émulsionnants, des polymères anioniques, cationiques, non-ioniques, amphotères ou leurs mélanges, des anti-perspirants, des agents alcalinisants, des colorants, des pigments, des agents propulseurs, des anti-oxydants et des anti-radicaux libres.

7. Composition cosmétique comportant au moins un composé de formule (I) :
R_{F} - (CH₂)ₙ -X - [C₃H₅(OH)] - Y - R_{H} (I)
dans laquelle C₃H₅(OH) représente les structures : 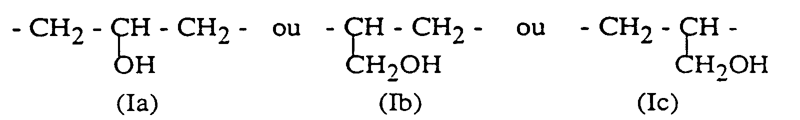
R_{F} représente un radical alkyle perfluoré en C₄-C₂₀ ou un mélange de radicaux alkyle perfluorés en C₄-C₂₀;
R_{H} représente un radical alkyle linéaire ou ramifié en C₁-C₂₂ ou un mélange de radicaux alkyle linéaires ou ramifiés en C₁-C₂₂ ou un radical aryle ou aralkyle;
n est compris entre 0 et 4;
X et Y représentent S, 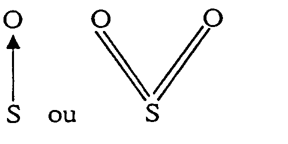
et au moins un adjuvant cosmétique.

8. Composition cosmétique selon la revendication 7, caractérisée en ce qu'elle comporte un ou plusieurs adjuvants cosmétiques choisis dans le groupe formé des corps gras, des solvants organiques, des silicones, les épaississants, les adoucissants, des filtres solaires UV-A ou UV-B ou à bande large, des agents anti-mousses, des agents hydratants, des humectants, des parfums, des conservateurs, des tensio-actifs, des charges, des séquestrants, des émulsionnants, des polymères anioniques, cationiques, non-ioniques amphotères ou leurs mélanges, des anti-perspirants, des agents alcalinisants, des colorants, des pigments, des agents propulseurs, des anti-oxydants et des anti-radicaux libres.

9. Composition cosmétique selon la revendication 7 ou 8, caractérisée en ce qu'elle est sous forme de lait, crème, lotion huileuse ou oléoalcoolique, gel gras ou oléoalcoolique, dispersion vésiculaire à base de lipides amphiphiles ioniques ou non-ioniques, bâtonnet solide, pâte, spray ou mousse aérosol.

10. Composition cosmétique selon l'une des revendications 7 à 9, caractérisée en ce qu'elle est sous forme de lait, de crème de soin pour la peau ou les cheveux, de crème, lotion ou lait démaquillant, de crème, gel, lotion ou lait anti-solaire, de crème ou mousse de rasage, de lotion après-rasage, de shampooing ou d'après-shampooing, de déodorant corporel, de pâte dentifrice, de laque, de produit de maquillage des cils, sourcils, ongles, lèvres ou peau, de crème de traitement de l'épiderme, de fond de teint, de bâton de rouge à lèvres, de fard à paupières ou à joues, d'eye-liner, mascara ou de produit de soin pour les lèvres ou les ongles.

11. Composition cosmétique selon l'une des revendications 7 à 10, caractérisée en ce qu'elle comporte 0,1 à 25% en poids du composé de formule (I).

12. Composés de formule :
R_{F} - (CH₂)ₙ - X - [C₃H₅(OH)] - Y - R_{H} (I')
dans laquelle C₃H₅(OH) représente les structures : 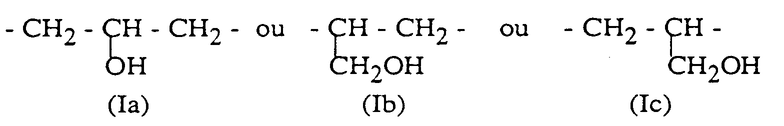
R_{F} représente un radical alkyle perfluoré en C₄-C₂₀ ou un mélange de radicaux alkyle perfluorés en C₄-C₂₀;
R_{H} représente un radical alkyle linéaire ou ramifié en C₁-C₂₂ ou un mélange de radicaux alkyle linéaires ou ramifiés en C₁-C₂₂ ou un radical aryle ou aralkyle;
n est compris entre 0 et 4;
X et Y représentent

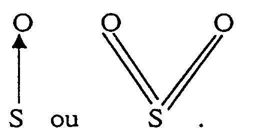

13. Composés selon la revendication 12, caractérisés en ce que :
R_{F} représente un radical alkyle perfluoré en C₆-C₁₂;
R_{H} représente un radical alkyle linéaire ou ramifié en C₃-C₁₈, un radical aryle en C₆-C₁₀ ou un radical aralkyle en C₇-C₁₅, et
n est 2.

## Claims

1. Use for preparing cosmetic compositions of compounds with formula :
R_{F} - (CH₂)ₙ - X - [C₃H₅(OH)] - Y - R_{H} (I)
where
C₃H₅(OH) represents the structures: 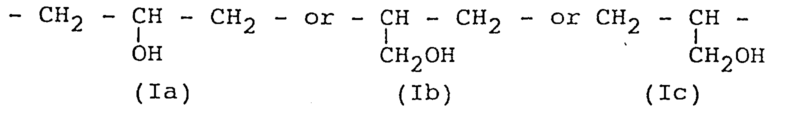
R_{F} represents a perfluorinated C₄-C₂₀ alkyl radical or a mixture of perfluorinated C₄-C₂₀ alkyl radicals;
R_{H} represents a linear or branched C₁-C₂₂ alkyl radical or a mixture of linear or branched C₁-C₂₂ alkyl radicals, an aryl radical or an aralkyl radical;
n is between 0 and 4;
X and Y represent S,

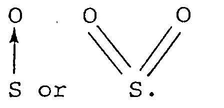

2. Use according to claim 1 characterized in that in formula (I),
R_{F} represents a perfluorinated C₆-C₁₂ alkyl radical,
R_{H} represents a linear or branched C₃-C₁₈ alkyl radical, a C₆-C₁₀ aryl radical or a C₇-C₁₅ aralkyl radical,
n equals 2, and
X and Y represent S.

3. Use according to claim 1 or claim 2 characterized in that the cosmetic composition is in the form of a milk, cream, oily or oleoalcoholic lotion, oily or oleoalcoholic gel, ionic or non-ionic amphiphilic lipid based vesicular dispersion, solid stick, paste, spray or aerosol foam.

4. Use according to any one of claims 1 to 3 characterized in that the cosmetic composition is in the form of a milk, a skin or hair cream, a make-up removing cream, lotion or milk, a sun protecting cream, gel, milk or lotion, a shaving cream or foam, an aftershave lotion, a shampoo or conditioner, a body deodorant, a toothpaste, a lacquer, a hair dye, a hair perming composition, an eyelid, eyelash, nail, lip or skin make-up, a skin treatment cream, a foundation, a lipstick, an eye-shadow, a blusher, an eyeliner, a mascara, a lip care product or a nail care product.

5. Use according to any one of claims 1 to 4 characterized in that the cosmetic composition comprises 0.1% to 25% by weight of the compound with formula (I).

6. Use according to any one of claims 1 to 5 characterized in that the cosmetic composition comprises one or more additives selected from the group formed by the normal fatty substances, organic solvents, silicones, thickeners, softeners, UV-A or UV-B or broad spectrum solar filters, anti-foaming agents, moisturising agents, humectants, fragrances, preservatives, surfactants, fillers, sequestrating agents, emulsifiers, anionic, cationic, non-ionic or amphoteric polymers and their mixtures, antiperspirants, alkalinizing agents, dyes, pigments, propellants, anti-oxidizing agents and free radical absorbers.

7. cosmetic composition comprising at least one compound with formula (I):
R_{F} - (CH₂)ₙ - X - [C₃H₅(OH)] - Y - R_{H} (I)
where
C₃H₅(OH) represents the structures: 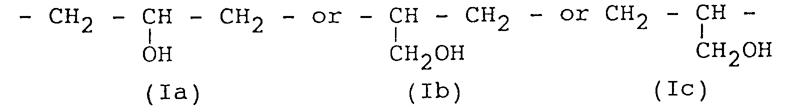
R_{F} represents a perfluorinated C₄-C₂₀ alkyl radical or a mixture of perfluorinated C₄-C₂₀ alkyl radicals;
R_{H} represents a linear or branched C₁-C₂₂ alkyl radical or a mixture of linear or branched C₁-C₂₂ alkyl radicals, an aryl radical or an aralkyl radical;
n is between 0 and 4;
X and Y represent S, 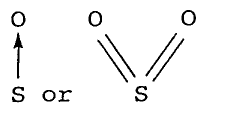
and at least one cosmetic additive.

8. A cosmetic composition according to claim 7 characterized in that it comprises one or more additives selected from the group formed by fatty substances, organic solvents, silicones, thickeners, softeners, UV-A or UV-B or broad spectrum solar filters, anti-foaming agents, moisturising agents, humectants, fragrances, preservatives, surfactants, fillers, sequestrating agents, emulsifiers, anionic, cationic, non-ionic or amphoteric polymers and their mixtures, antiperspirants, alkalinizing agents, dyes, pigments, propellants, anti-oxidizing agents and free radical absorbers.

9. A cosmetic composition according to claim 7 or claim 8 characterized in that it is in the form of a milk, cream, oily or oleoalcoholic lotion, oily or oleoalcoholic gel, ionic or non-ionic amphiphilic lipid based vesicular dispersion, solid stick, paste, spray or aerosol foam.

10. A cosmetic composition according to any one of claims 7 to 9 characterized in that it is in the form of a milk, a skin or hair cream, a make-up removing cream, lotion or milk, a sun protecting cream, gel, milk or lotion, a shaving cream or mousse, an aftershave lotion, a shampoo or conditioner, a body deodorant, a toothpaste, a lacquer, an eyelid, eyelash, nail, lip or skin make-up, a skin treatment cream, a foundation, a lipstick, an eye-shadow, a blusher, an eyeliner, a mascara, a lip care product or a nail care product.

11. A cosmetic composition according to any one of claims 7 to 10 characterized in that it comprises 0.1% to 25% by weight of the compound with formula (I).

12. A compound with formula:
R_{F} - (CH₂)ₙ - X - [C₃H₅(OH)] - Y - R_{H} (I')
where
C₃H₅(OH) represents the structures: 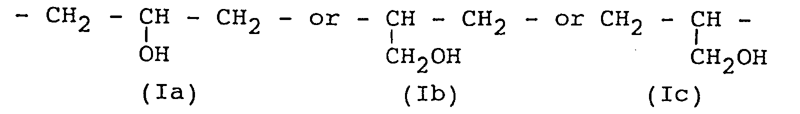
R_{F} represents a perfluorinated C₄-C₂₀ alkyl radical or a mixture of perfluorinated C₄-C₂₀ alkyl radicals;
R_{H} represents a linear or branched C₁-C₂₂ alkyl radical or a mixture of linear or branched C₁-C₂₂ alkyl radicals, an aryl radical or an aralkyl radical;
n is between 0 and 4;
X and Y represent

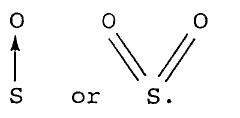

13. A compound according to claim 12 characterized in that:
R_{F} represents a perfluorinated C₆-C₁₂ alkyl radical;
R_{H} represents a linear or branched C₃-C₁₈ alkyl radical, a C₆-C₁₀ aryl radical or a C₇-C₁₅ aralkyl radical; and
n equals 2.

## Patentansprüche

1. Verwendung der Verbindungen der Formel:
R_{F} - (CH₂)ₙ - X - [C₃H₅(OH)] - Y - R_{H} (I)
worin C₃H₅(OH) die Strukturen darstellt: 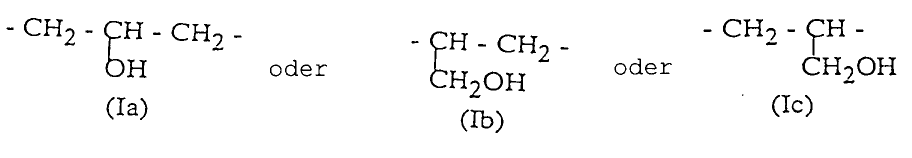
und worin R_{F} einen perfluorierten C₄₋₂₀-Alkylrest oder eine Mischung perfluorierter C₄₋₂₀-Alkylreste,
R_{H} einen linearen oder verzweigten C₁₋₂₂-Alkylrest oder eine Mischung linearer oder verzweigter C₁₋₂₂-Alkylreste oder einen Aryl- oder Aralkylrest darstellen,
n 0 bis 4 beträgt und
X und Y S, 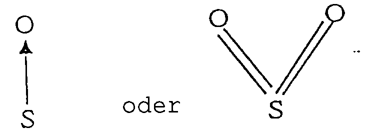 darstellen,
zur Herstellung kosmetischer Zusammensetzungen.

2. Verwendung gemaß Anspruch 1,
dadurch **gekennzeichnet**, daß
in Formel (I)
R_{F} einen perfluorierten C₆₋₁₂-Alkylrest,
R_{H} einen linearen oder verzweigten C₃₋₁₈-Alkylrest, einen C₆₋₁₀-Arylrest oder einen C₇₋₁₅-Aralkylrest darstellen,
n 2 ist und
X und Y S darstellen.

3. Verwendung gemäß Anspruch 1 oder 2,
dadurch **gekennzeichnet**, daß
die kosmetischen Zusammensetzungen in Form einer Milch, Creme, öligen oder oleoalkoholischen Lotion, eines fetten oder oleoalkoholischen Gels, einer bläschenartigen Dispersion auf Basis amphiphiler ionischer oder nicht-ionischer Lipide, eines Feststoffstäbchens, einer Paste, eines Spray oder Aerosol-Schaums vorliegen.

4. Verwendung gemäß einem der Ansprüche 1 bis 3,
dadurch **gekennzeichnet**, daß
die kosmetischen Zusammensetzungen in Form einer Milch, Creme zur Pflege der Haut oder der Haare, Creme, Lotion oder Milch zum Entschminken, Creme, eines Gels, einer Lotion oder Milch als Sonnenschutzmittel, Creme oder eines Schaums zur Rasur, Lotion zur Anwendung nach der Rasur, eines Shampoo oder Nach-Shampoo, eines Körperdeodorant, einer Zahnpaste, eines Lacks, einer Zusammensetzung zur Haarfärbung, einer Zusammensetzung zur Dauerwellengebung der Haare, eines Produkts zum Schminken der Wimpern, Augenbrauen, Nägel, Lippen oder der Haut, einer Creme zur Behandlung der Epidermis, einer Teint-Grundlage, eines Lippenstifts, einer Schminke für Augenlider oder Wangen, eines Liniermittels für die Augen, einer Wimperntusche oder eines Produkts zur Pflege der Lippen oder Nägel vorliegen.

5. Verwendung gemäß einem der Ansprüche 1 bis 4,
dadurch **gekennzeichnet**, daß
die kosmetischen Zusammensetzungen 0,1 bis 25 Gew.% der Verbindung der Formel (I) enthalten.

6. Verwendung gemäß einem der Ansprüche 1 bis 5,
dadurch **gekennzeichnet**, daß
die kosmetischen Zusammensetzungen einen oder mehrere Hilfsstoffe enthalten, ausgewählt aus der Gruppe aus üblichen Fettkörpern, organischen Lösungsmitteln, Siliconen, Verdickungsmitteln, weichmachenden Mitteln, Sonnenfilterstoffen für UV-A oder UV-B oder für den langwelligen Bandenbereich, Antischaummitteln, hydratisierenden Mitteln, Feuchtigkeitsmitteln, Parfüm-Produkten, Konservierungsstoffen, oberflächenaktiven Mitteln, Beaufschlagungsmittel, Sequestriermittel, Emulgatoren, anionischen, kationischen, nicht-ionischen und amphoteren Polymeren oder deren Mischungen, Antischweißmitteln, alkalisch stellenden Mitteln, Farbstoffen, Pigmenten, Treibmitteln, Antioxidantien und aus Abfangmitteln für freie Radikale.

7. Kosmetische Zusammensetzung, enthaltend mindestens eine Verbindung der Formel (I):
R_{F} - (CH₂)ₙ - X - [C₃H₅(OH)] - Y - R_{H} (I)
worin C₃H₅(OH) die Strukturen darstellt: 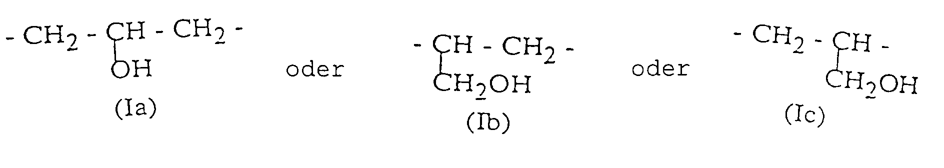
und worin R_{F} einen perfluorierten C₄₋₂₀-Alkylrest oder eine Mischung perfluorierter C₄₋₂₀-Alkylreste,
R_{H} einen linearen oder verzweigten C₁₋₂₂-Alkylrest oder eine Mischung linearer oder verzweigter C₁₋₂₂-Alkylreste oder einen Aryl- oder Aralkylrest darstellen,
n 0 bis 4 beträgt und
X und Y S, 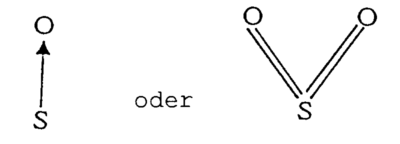 darstellen,
und enthaltend mindestens einen kosmetischen Hilfsstoff.

8. Kosmetische Zusammensetzung gemäß Anspruch 7,
dadurch **gekennzeichnet**, daß
sie einen oder mehrere kosmetische Hilfsstoffe enthält, ausgewählt aus der Gruppe aus Fettkörpern, organischen Lösungsmitteln, Siliconen, Verdickungsmitteln, weichmachenden Mitteln, Sonnenfilterstoffen für UV-A oder UV-B oder für den langwelligen Bandenbereich, Antischaummitteln, hydratisierenden Mitteln, Feuchtigkeitsmitteln, Parfüm-Produkten, Konservierungsstoffen, oberflächenaktiven Mitteln, Beaufschlagungsmittel, Sequestriermittel, Emulgatoren, anionischen, kationischen, nicht-ionischen und amphoteren Polymeren oder deren Mischungen, Antischweißmitteln, alkalisch stellenden Mitteln, Farbstoffen, Pigmenten, Treibmitteln, Antioxidantien und aus Abfangmitteln für freie Radikale.

9. Kosmetische Zusammensetzung gemäß Anspruch 7 oder 8,
dadurch **gekennzeichnet**, daß
sie in Form einer Milch, Creme, öligen oder oleoalkoholischen Lotion, eines fetten oder oleoalkoholischen Gels, einer bläschenartigen Dispersion auf Basis amphiphiler ionischer oder nicht-ionischer Lipide, eines Feststoffstäbchens, einer Paste, eines Spray oder Aerosolschaums vorliegt.

10. Kosmetische Zusammensetzung nach einem der Ansprüche 7 bis 9,
dadurch **gekennzeichnet**, daß
sie in Form einer Milch oder Creme zur Pflege der Haut oder der Haare, Creme, Lotion oder Milch zum Entschminken, Creme, eines Gels, einer Lotion oder Milch als Sonnenschutzmittel, Creme oder eines Schaums zur Rasur, Lotion zur Anwendung nach der Rasur, eines Shampoo oder Nach-Shampoo, eines Körperdeodorant, einer Zahnpaste, eines Lacks, einer Zusammensetzung zur Haarfärbung, einer Zusammensetzung zur Dauerwellengebung der Haare, eines Produkts zum Schminken der Wimpern, Augenbrauen, Nägel, Lippen oder der Haut, einer Creme zur Behandlung der Epidermis, einer Teint-Grundlage, eines Lippenstifts, einer Schminke für Augenlider oder Wangen, eines Liniermittels für die Augen, einer Wimperntusche oder eines Produkts zur Pflege der Lippen oder Nägel vorliegen.

11. Kosmetische Zusammensetzung gemäß einem der Ansprüche 7 bis 10,
dadurch **gekennzeichnet**, daß
sie 0,1 bis 25 Gew.% der Verbindung der Formel (I) enthält.

12. Verbindungen der Formel:
R_{F} - (CH₂)ₙ - X - [C₃H₅(OH)] - Y - R_{H} (I')
worin C₃H₅(OH) die Strukturen darstellt: 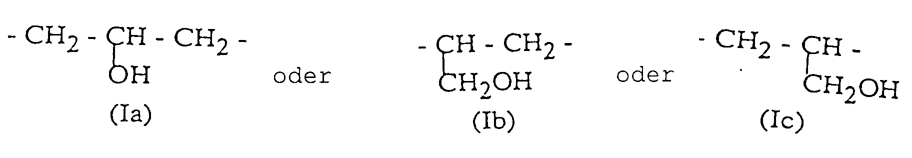
und worin R_{F} einen perfluorierten C₄₋₂₀-Alkylrest oder eine Mischung perfluorierter C₄₋₂₀-Alkylreste,
R_{H} einen linearen oder verzweigten C₁₋₂₂-Alkylrest oder eine Mischung linearer oder verzweigter C₁₋₂₂-Alkylreste oder einen Aryl- oder Aralkylrest darstellen,
n 0 bis 4 beträgt und
X und Y 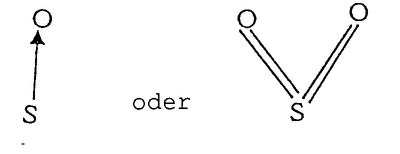 darstellen.

13. Verbindungen gemäß Anspruch 12,
dadurch **gekennzeichnet**, daß gilt:
R_{F} stellt einen perfluorierten C₆₋₁₂-Alkylrest dar;
R_{H} stellt einen linearen oder verzweigten C₃₋₁₈-Alkylrest, einen C₆₋₁₀-Arylrest oder einen C₇₋₁₅-Aralkylrest dar, und
n ist 2.
